# EUROPEAN PATENT SPECIFICATION

(11) **EP 0 646 176 B1**
(45) Date of publication and mention of the grant of the patent: **23.06.2004**
(21) Application number: 93908349.9
(22) Date of filing: 12.03.1993
(51) Int. Cl.: C12N 15/62, C12N 15/31, A61K 38/00, A61K 39/02, A61K 39/12, C12N 15/74, C12N 15/90, A61K 38/46, A61K 39/35

(54) **DELIVERY AND EXPRESSION OF A HYBRID SURFACE PROTEIN ON THE SURFACE OF GRAM POSITIVE BACTERIA**
TRANSPORT UND EXPRESSION EINES HYBRIDEN OBERFLÄCHENPROTEINS AN DER OBERFLÄCHE VON GRAMPOSITIVEN BAKTERIEN
APPORT ET EXPRESSION D'UNE PROTEINE DE SURFACE HYBRIDE SUR LA SURFACE DE BACTERIES GRAM POSITIF

(30) Priority: 13.03.1992 US 851082
(43) Date of publication of application: 05.04.1995
(73) Proprietor: THE ROCKEFELLER UNIVERSITY, New York, NY 10021 (US)
(72) Inventor: FISCHETTI, Vincent, A., West Hempstead, NY 11552 (US); POZZI, Gianni, I-53018 Sovicille (IT); SCHNEEWIND, Olaf, Los Angeles, CA 90024 (US)
(74) Representative: Fagerlin, Heléne
(86) International application number: PCT/US1993/002355
(87) International publication number: WO 1993/018163

(56) References cited:
- WO-A-90/15872
- WO-A-92/20805
- ZENTRALBLATT FUER BAKTERIOLOGIE SUPPLEMENT, 22 (INTERNATIONAL JOURNAL OF MEDICAL MICROBIOLOGY) 1992, GUSTAV FISCHER VERLAG;STUTTGART,BRD; OREFICI, G. (ED.). 'NEW PERSPECTIVES ON STREPTOCOCCI AND STREPTOCOCCAL INFECTIONS'
- XI lancefield international symposium on Streptococci and streptococcal diseases, Siena, Italy, September 10-14, 1990; p 350- 352; the whole document
- BIO/TECHNOLOGY vol. 9, no. 12, December 1991, NATURE AMERICA, INC., NEW YORK, US pages 1369 - 1372 P. FUCHS ET AL. 'Targeting recombinant antibodies to the surface of Escherichia coli: Fusion to a peptidoglycan associated lipoprotein'
- GENE vol. 104, no. 2, 15 August 1991, ELSEVIER SCIENCE, AMSTERDAM, NL; pages 147 - 153 F. BREITLING ET AL. 'A surface expression vector for antibody screening'
- SCIENCE vol. 244, 7 April 1989, AAAS, WASHINGTON, DC, US; pages 70 - 72 S.M.C. NEWTON ET AL. 'Immune response to cholera toxin epitope inserted in Salmonella flagellin'
- NATURE vol. 351, 6 June 1991, MACMILLAN JOURNALS LTD,LONDON,UK; pages 456 - 460 C.K. STOVER ET AL. 'New use of BCG for recombinant vaccines' cited in the application the whole document
- INFECTION AND IMMUNITY vol. 60, no. 5, May 1992, AM.SOC.MICROBIOL.,WASHINGTON,DC,US; pages 1902 - 1907 G. POZZI ET AL. 'Delivery and expression of a heterologous antigen on the surface of streptococci'
- SCHNEEWIND ET AL: 'Surface proteins from gram-positive cocci have a common motif for membrane anchoring' GENETICS AND MOLECULAR BIOLOGY OF STREPTOCOCCI, LACTOCOCCI AND ENTEROCOCCI 1991, pages 152 - 154
- FISCHETTI ET AL: 'Conservation of a hexapeptide sequence in the anchor region of surface proteins from gram-positive cocci' MOLECULAR MICROBIOLOGY vol. 4, no. 8, 1990, pages 1603 - 1605

## Description

### RELATED APPLICATIONS

This application is a continuation in part of copending application serial number 07/851,082 filed March 13, 1992 which is a continuation in part of application serial number 07/814,823 filed December 23, 1991 which is, a continuation of application serial number 07/742,199 filed August 5, 1991 which is, in turn, a continuation of application serial number 07/522,440 filed May 11, 1990. The latter two applications are now abandoned.

### FIELD OF THE INVENTION

This invention relates generally to products and processes useful to deliver proteins, including protein antigens, to the surface of gram-positive bacteria and firmly attach them to the cell. More specifically, it relates to the production of a fusion protein containing at least the anchor region of the cell associated region of a gram-positive surface protein and any protein, peptide or polypeptide that may be usefully administered to an animal host. The products of the invention which comprise proteins, peptides or polypeptides, as further discussed below, placed on the surface of a gram positive bacteria may be used to deliver such materials to the animal host to elicit an immungenic response, for example the production of protective antibodies or for another useful purpose. The protein, peptide or polypeptide may, for example, be an enzyme or other functional protein necessary for a specific purpose. It may be an antigenic determinant from a bacteria, virus, parasite or fungus. It may be a surface antigen from a mammalian tumor cell or of male sperm. It may be an allergen such as a vespid venom. The invention also relates to novel plasmids, genes, chromosomes and transformed bacteria employed in the production of such fusion proteins. The invention, additionally, provides novel vaccines which employ gram-positive bacteria designed to deliver to an animal host a foreign antigen normally present on a pathogenic microorganism which is associated with the virulence of that pathogen and which will elicit antigens to protect the host against infection or disease caused by the pathogenic microorganism.

The products of the invention are also useful as diagnostic agents.

The invention also provides a means to deliver enzymes, placed on the bacterial surface, to specific areas of interest.

The term "animal" as used herein refers to living beings including mammals such as man; bovines, especially beef cattle; sheep and goats; poultry, especially chickens, ducks and turkeys; as well as fish, especially those raised in fish farms such as salmon, trout and catfish. This invention is of special importance to mammals.

### BACKGROUND OF THE INVENTION

The essence of this invention is that it provides a method for the production of novel non-pathogenic gram positive bacteria expressing a hybrid surface protein which may be a hybrid surface antigen, comprising two principal parts, an anchor segment comprised of amino acid residues and an N-terminal active polypeptide segment, both of which will be defined and discussed in more detail below.

This invention will be better understood by consideration of the M protein and its structure. The M protein is a coiled coil surface antigen which is the virulence factor of group A streptococci, a gram positive bacteria. The M protein of Streptococcus pyogenes of M type 6 contains 441 amino acid residues.

Its structure will be discussed in more detail below, but it will be useful to discuss the cell associated region at this point. Using the standard one letter representation of amino acids, the structure of the anchor region of the cell associated region of the M6 protein from amino acid residue 407 to residue 441 may be represented as: LPSTGETANPFFTAAALTVMATAGVAAVVKRKEEN Reading from the first leucine residue (L) at the N-terminal of the anchor region, the region includes the LPSTGE segment; a spacer segment containing three amino acid residues TAN; a hydrophobic segment of twenty amino acids, PFFTAAALTVMATAGVAAVV: followed by a highly charged tail segment, KRKEEN.

It has been observed as a result of structural studies of a large number of surface proteins of gram positive bacteria that the above described anchor region of the M6 protein is highly conserved among all known surface proteins of gram positive bacteria. Many of them are shown by Fischetti et al (Reference 1). Generally, the hydrophobic segment contains about 15 to 20 amino acid residues, the charged tail segment about 4 to 6 amino acid residues, and the spacer segment from about 3 to 6 amino acid residues. Most remarkable however, is the high degree of homology, practically 100% in the LPSTGE segment of the known surface proteins. The variations that occur are almost exclusively at the 3 and 6 positions. Therefore, the region may be generally represented as LPXTGX.

The following Table 1 shows the remarkable extent of this homology thus far established amongst forty different surface proteins of gram positive bacteria.

**TABLE 1**

| SEQUENCED SURFACE PROTEINS FROM GRAM-POSITIVE BACTERIA | | | | | |
|---|---|---|---|---|---|
| NAME/GENE | | SURFACE PROTEIN | ORGANISM | LPSTGE | REF |
| 1. | M6 | M protein | S. pyogenes | LPSTGE | (2) |
| 2. | M5 | M protein | S. pyogenes | LPSTGE | (3) |
| 3. | M12 | M protein | S. pyogenes | LPSTGE | (4) |
| 4. | M24 | M protein | S. pyogenes | LPSTGE | (5) |
| 5. | M49 | M protein | S. pyogenes | LPSTGE | (6) |
| 6. | M57 | M protein | S. pyogenes | LPSTGE | (7) |
| 7. | M2 | M protein | S. pyogenes | LPSTGE | (8) |
| 8. | ARP2 | IgA binding protein | S. pyogenes | LPSTGE | (8) |
| 9. | ARP4 | IgA binding protein | S. pyogenes | LPSTGE | (9) |
| 10. | FcRA | Fc binding protein | S. pyogenes | LPSTGE | (10) |
| 11. | Prot H | Human IgG Fc binding | S. pyogenes | LPSTGE | (11) |
| 12. | SCP | C5a peptidase | S. pyogenes | LPTTND | (12) |
| 13. | T6 | Protease resistant protein | S. pyogenes | LPSTGS | (13) |
| 14. | bac | IgA binding protein | Gr. B strep | LPYTGV | (14) |
| 15. | Prot G | IgG binding protein | Gr. G strep | LPTTGE | (15) |
| | | | | | |
| 16. | PAc | Surface protein | S. mutans | LPNTGE | (16) |
| 17. | spaP | Surface protein | S. mutans | LPNTGE | (17) |
| 18. | spaA | Surface protein | S. sobrinus | LPATGD | (18) |
| 19. | wapA | Wall-associated protein A | S. mutans | LPSTGE | (19) |
| | | | | | |
| 20. | Sec10 | Surface protein | E. fecalis | LPQTGE | (20) |
| 21. | Asc10 | Surface protein | E. fecalis | LPKTGE | (20) |
| 22. | asa1 | Aggregation substance | E. fecalis | LPQTGE | (21) |
| 23. | Prot A | IgG binding protein | S. aureus | LPETGV | (22) |
| 24. | FnBP | Fibronectin binding protein | S. aureus | LPETGG | (23) |
| 25. | wg2 | Cell wall protease | S. cremoris | LPKTGE | (24) |
| | | | | | |
| 26. | InlA | Internalization protein | L. monocytogenes | LPTTGE | (25) |
| 27. | Fimbriae | Type 1 fimbriae | A. viscosis | LPLTGA | (26) |
| 28. | Fimbriae | Type 2 fimbriae | A. naeslundii | LPLTGA | (27) |
| 29. | Mrp4 | IgG/Fibrinogen binding | S. pyogenes | LPSTGE | (10) |
| 30. | sof22 | Serum opacity factor | S. pyogenes | LPASGD | (54) |
| 31. | Sfb | Fibronectin binding | S. pyogenes | LPATGD | (55) |
| 32. | Prot L | Light chain binding | P. magnus | LPKAGS | (56) |
| 33. | bca | alpha antigen | Gr. B strep | LPATGE | (57) |
| 34. | fnbA | Fibronectin binding | S. dysgalactiae | LPQTGT | (58) |
| 35. | fnbB | Fibronectin binding | S. dysgalactiae | LPAAGE | (59) |
| 36. | EmmGl | M protein | Gr. G Strep | LPSTGE | (60) |
| 37. | DG12 | Albumin binding protein | Gr. G strep | LPSTGE | (61) |
| 38. | MRP | Surface protein | S. suis | LPNTGE | (62) |
| 39. | FnBp | Fibronectin binding protein | S. aureus | LPETGG | (63) |
| 40. | cna | Collagen binding protein | S. aureus | LPKTGM | (64) |

It is apparent that this highly homologous region of the surface proteins of gram-positive bacteria is essential to anchoring bacterial surface proteins to the cell (28). This segment, which is referred to herein as the LPXTGX segment, is the crucial segment of the cell associated region of surface protein for anchoring the proteins to the surface of gram positive bacteria.

This discovery has been confirmed by Schneewind et al (65). Using the Protein A molecule of Staphlococcus aureus, these investigators have established that the complete complex (LPXTGX motif, hydrophobic domain and charged tail) are necessary to deliver the Protein A molecule to the cell surface and that changes in the LPXTGX motif or deletion thereof will not inhibit expression of the molecule but will prevent it from anchoring to the surface.

### BRIEF DESCRIPTION OF THE DRAWINGS

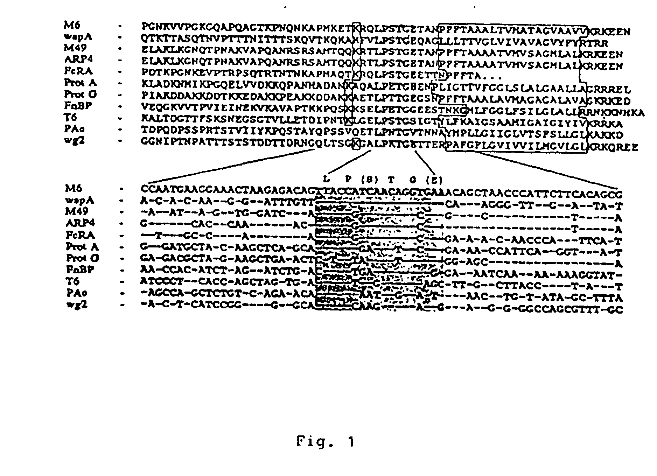

Fig. 1 shows the alignment of the amino acids in the C-terminal regions of a variety of surface antigens from gram-positive bacteria and the gene segments which are employed to express them. The LPSTGE motif is shaded and the proteins were aligned along this consensus sequence. In 10 out of 11 proteins, a conserved lysine residue was found 2 or 3 residues preceding the consensus LPSTGE sequence (boxed). The homologous carboxyterminal hydrophobic regions are also boxed. Abbreviations used in the left column are the same as those in Table 1.
Fig. 2 a model of the M protein.
Fig. 3 shows the complete amino acid sequence of the M6 protein.
Fig. 4 shows the host vector system GP232-pVMB20.
Fig. 5 shows the construction and chromosomal integration of the M6:E7 translation fusion in GP246.
Fig. 6 shows the results of studies designed to demonstrate that the M6:E7 fusion protein expressed in Streptococcus gordonii GP246 located on its surface.
Fig. 7 shows that cell extracts of recombinant Streptococcus gordonii 246 expresses the M6:E7 fusion protein.
Fig. 8 shows that animals immunized with the recombinant GP246 produce antibodies reactive with the E7 protein.
Fig. 9 shows a universal plasmid of the invention.
Fig. 10 shows a typical hybrid protein of the invention.
Figs. 11 and 12 show the results of studies in which mice were treated with a hybrid protein of the invention.

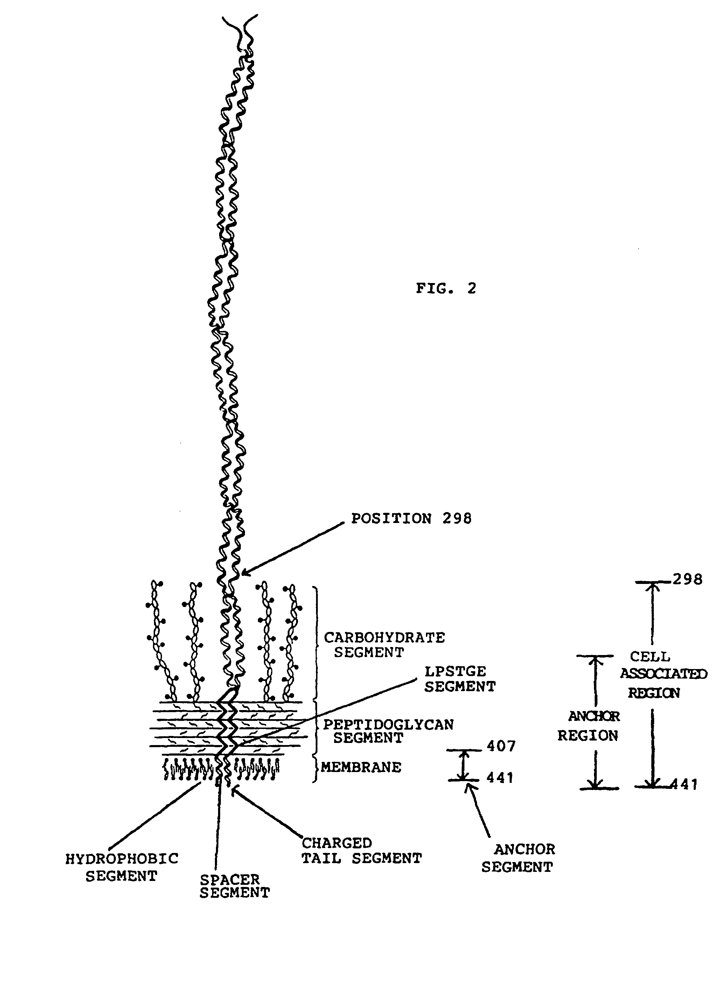

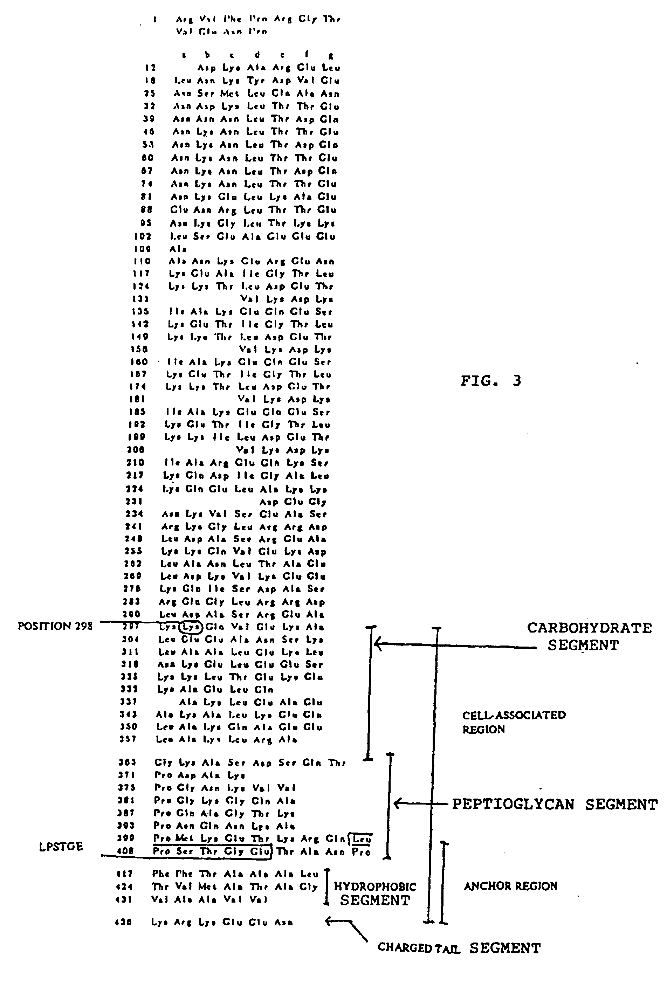

Careful study of Figs 2 and 3 will assist in understanding this invention. Fig. 2 represents a model of the M protein with certain of the positions and segments identified. Fig. 3 identifies each amino acid residue in the complete structure of the M6 protein.

It will be understood from the foregoing discussion that the segment of the M protein identified as the cell associated region in Fig. 2 is analogous to regions for surface proteins found on other gram-positive bacteria such as those listed in Table 1. As discussed above, there is a high degree of homology particularly within the anchor sequence especially the LPXTGX segment found in all gram-positive surface proteins. As will be described below, and for the purpose of illustrating this invention, the gene segment spanning region 122 to 300 of the gene which expresses the M protein is genetically removed and replaced by a new gene segment expressing a foreign protein, for example, an antigen which will generate useful antibodies, thereby producing a novel hybrid surface protein. Because of the great degree of homology within the cell associated regions of all gram-positive bacteria, the hybrid gene can be employed in any gram positive bacteria. The selected bacteria will express the designed hybrid protein using the anchor region to attach the molecule to the cell and positioning the inserted active segment on the cell surface. The inserted active segment is hereinafter referred to as the "active polypeptide".

The term "active polypeptide" is used herein in the broadest possible sense. It refers to any peptide, polypeptide or protein which may be delivered to an animal host for any useful purpose. For example, as described in detail hereinafter, the cell associated region of a protein from gram-positive bacteria can be fused to a segment of a viral protein from a pathogen to produce a hybrid surface protein which will be expressed by non-pathogenic bacteria. The bacteria can colonize an animal host and function as a vaccine. It will elicit antibodies in an animal host to protect against or inhibit subsequent infection by the virus. The fused viral segment is the "active polypeptide" of that particular embodiment of the invention.

For convenience, the term "polypeptide" will hereinafter be used to refer to molecules which are of sufficiently high molecular weight to be called proteins, to products of lesser molecular weight, usually called polypeptides, and to products of even lesser molecular weights normally referred to as peptides.

The "active polypeptide" may be any polypeptide which can be delivered to an animal host for a useful purpose. It could be, for example the viral segment referred to above. It could also be an antigen, from any pathogenic virus, or from a bacteria, parasite or fungi. In such instances, the active polypeptide may be the complete antigen, the antigenic determinant of the antigen or a segment of the antigen which includes the antigenic determinant. The useful purpose will be to elicit a protective immune response by the production of antibodies or to elicit a cellular immune response to the antigen.

The term "cell associated region" as used in this specification and claims will be readily understood by reference to the figures, especially Figs. 2, 3 and 10. It will be seen that the cell associated region includes a cell wall spanning sequence of amino acid residues and a carbohydrate spanning segment. The wall spanning sequence may be omitted from the hybrid proteins of this invention, although it is presently preferred not to do so.

The cell associated region also includes the anchor region which contains the anchor segment (LPXTGX), the spacer segment, the hydrophobic segment and the charged tail segment. The anchor sequence is essential to the hybrid proteins of this invention. Without this sequence the hybrid protein will not be retained on the surface of the gram positive bacterial carrier.

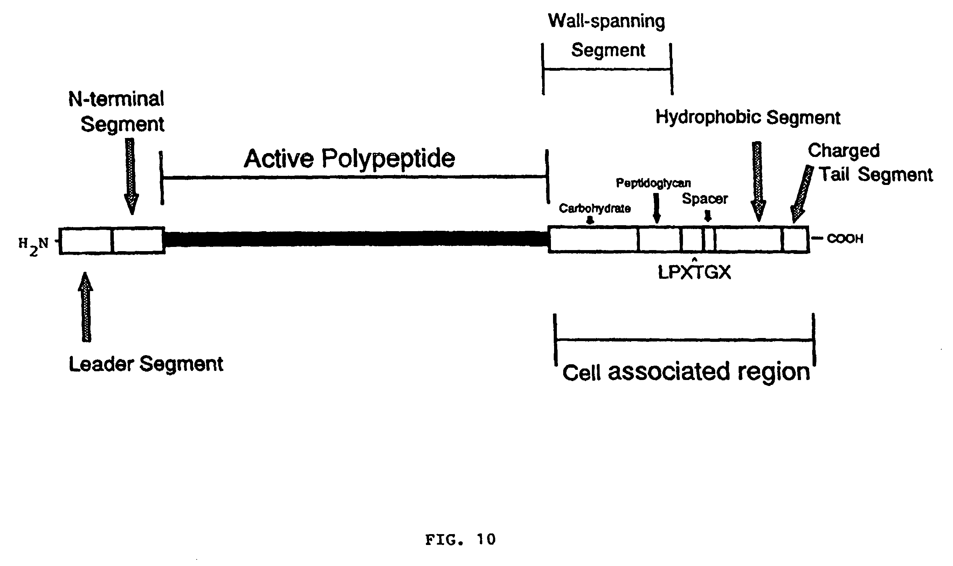

Fig. 10 shows the balance of a typical hybrid protein of the invention which, as shown, includes the active polypeptide fused to the cell associated region. The figure also shows a leader segment and a small N-terminal segment at the amino terminal of the active polypeptide. These segments cooperate in the proper placement of the hybrid protein on the surface of the gram positive bacteria. The function of the leader segment is to permit the proper processing of the hybrid protein within the cell. The N-terminal segment in cooperation with the leader segment enables the leader peptidase enzyme to separate the leader segment from the N-terminal segment and permit the hybrid protein to assume its proper position on the surface of the bacteria. The leader segment and the N-terminal segment are from the same surface protein as the protein of the cell associated region. The N-terminal segment suitably contains the first few amino acid residues derived from the amino end of the protein used in the cell associated region of the hybrid protein. About ten such residues are normally sufficient for the proper processing, but segments containing up to twenty or more amino acid residues may be employed.

The active polypeptide is not necessarily an antigen from a pathogenic organism. It may also be an enzyme, for example. In that event, the useful purpose will be to deliver the enzyme on the surface of a non-pathogenic bacteria to a specific site in the animal host colonized by that non-pathogenic bacteria.

The active polypeptide may be the complete molecule which comprises the enzyme. Alternatively, it may be only that segment of the polypeptide which is required to accomplish the useful purpose.

The active polypeptide may be an antigen which will react with antibodies elicited by pathogenic microorganisms. Bacteria of the invention carrying such active polypeptide antigens are useful as diagnostic reagents.

When reference is made herein to non-pathogenic bacteria it should be understood to include gram positive commensal bacteria as well as pathogenic bacteria which have been modified or attenuated by the usual procedures so that their pathogenicity is weakened or destroyed and which express hybrid proteins in accordance with the process described herein, such hybrid proteins containing a C-terminal region including the LPXTGX region.

### BRIEF DESCRIPTION OF THE INVENTION

This invention provides non-pathogenic gram positive bacteria which express at least one hybrid surface protein the C-terminal region of which is attached to the bacteria. The C-terminal region includes, but is not limited to, the cell associated region of a surface protein normally expressed by a gram-positive bacteria which may be pathogenic or non-pathogenic. The balance of the hybrid surface protein includes an active polypeptide which may be delivered to an animal host for any useful purpose. The surface protein "normally produced" by the bacteria of this invention refers to the whole protein produced by the bacteria before it is genetically altered in accordance with the process of this invention.

As is known from previous studies (29) and indicated in Figs 2 and 3, amino acids 298-441 of the M protein are buried within the cell whereas residues 1-297 are exposed on the surface of the bacterial cell. In accordance with this invention, by employing genetic manipulations described and illustrated herein, nearly all of the surface exposed region of the M protein may be removed and replaced by an active polypeptide that is linked to the C-terminal cell-associated region of the M protein to produce a hybrid surface protein of the invention. Because the cell associated region is essentially the same for all gram-positive surface molecules (Fig. 10), a smimilar strategy may be used whereby the cell associated region of any one of these molecules may be used as a delivery carrier or vehicle to deliver an active polypeptide from another source. The new gene employed to express the hybrid surface protein may be inserted into a plasmid by standard techniques and the plasmid used to transform an Escherichia coli or other vector. The E. coli thereafter will express the new fusion protein, i.e. the hybrid protein of this invention. The E. coli procedure is useful for the production of large amounts of hybrid protein which can be isolated from the periplasm of this gram negative organism. However, for most utilities of this invention, it is preferred to transform a gram positive organism for the production of the hybrid protein on the surface of the gram positive bacteria. The recombinant genes produced in accordance with the invention may be processed by any gram positive bacteria.

Alternatively, the newly constructed plasmid containing the fusion gene may be used to integrate the fusion gene into the chromosome of a gram-positive bacteria, for example the chromosome of Streptococcus mutans. The newly produced strain of Streptococcus mutans will thereafter produce the new hybrid protein of this invention by expressing it on the cell surface. This is the presently preferred procedure for producing the products of this invention.

Antigenic polypeptides from a wide variety of microorganisms may be employed as the active polypeptides of the invention. These include pathogenic microorganisms which infect man and animals. There follows a representative list of typical microorganisms which express polypeptides useful in the practice of this inventions. The transformed bacteria of the invention may be used to treat or prevent the diseases associated with infection by the microorganism.
Fungi: Candida albicans, Aspergillus fumigatus, Histoplasma capsulatum (all cause disseminating disease), microsporum canis (animal ringworm)
Parasitic Protozoa: Plasmodium falciparum (malaria), Trypanosoma cruzi (Sleeping sickness),
Spirochetes: Borrelia bergdorferi (Lyme disease), Treponema pallidum (syphilis), Borrelia recurrentis (recurring fever), Leptospira icterohaemorrhagiae (leptospirosis)
Bacteria: Neisseria gonorrhoeae (gonorrhoea), Staphylococcus aureus (endocarditis), Streptococcus pyogenes (rheumatic fever), Salmonella typhosa (salmonellosis), Hemophilus influenzae (influenza), Bordetella pertussis (whooping cough), Actinomyces israelii (actimomyosis), Streptococcus mutans (dental caries)
Streptococcus equi - Strangles (Horses), Streptococcus agalactiae (bovine mastitis), Streptococcus anginosus (canine genital infections)
Viruses: Human immunodeficiency virus (HIV), Polio virus, Influenza virus, Rabies virus, Herpes virus, Foot and Mouth Disease virus, Psittacosis virus, Paramyxovirus, Myxovirus, Coronovirus

In one embodiment of this invention, a non-pathogenic gram-positive bacteria that is a commensal organism for the host animal will be used to express the hybrid protein on its surface, by inserting the gene coding for the hybrid protein into the non-pathogenic gram-positive bacteria. If the fusion is with an active polypeptide that is an antigen from a pathogenic organism (bacterial, viral, parasitic, or fungal), and the resulting commensal bacteria expressing the hybrid antigen is administered to an animal host, there will be an immunological response by both humoral and cell mediated pathways. One possible immunological response is the production of antibodies, thereby effecting protection against infection by the pathogen. If the fusion is with an enzyme, the enzyme will be expressed on the surface of the commensal. A wide variety of active polypeptides may be delivered to the surface of gram positive bacteria as will be apparent to the skilled artisan from a study of this disclosure.

The genes, plasmids, chromosomes, and transformed bacteria of this invention are produced by the application of standard recombinant techniques well known to those skilled in the art. For example, an oligonucleotide or a restriction fragment coding for a viral coat protein, the virulence factor of a bacterial surface antigen (or, in fact, the complete antigen), an enzyme or other desired progenitor of a hybrid surface protein of the invention may be ligated to the gene which expresses the cell associated region of the M protein or other known surface protein from a gram positive bacteria such as those listed in Table 1. The resulting hybrid gene will be used to transform a gram positive bacteria to effect the production of a surface hybrid protein of the invention.

This invention will be further explained in conection with the expression of the hybrid protein M6:E7 by the GP246 strain of Streptococcus gordonii. This hybrid antigen contains the C-terminal cell associated segment of the M6 protein. The antibody eliciting polypeptide, E7, is an early protein from a papillomavirus strain.

Streptococcus gordonii is a commensal bacteria of the oral cavity. E7 is a 98 amino acid early protein produced during viral replication of human papillomavirus type 16 (HPV16). It is an oncoprotein, antibodies to which are found in patients with cervical cancer. It is considered a major candidate antigen for vaccines against HPV-induced malignant neoplasias.

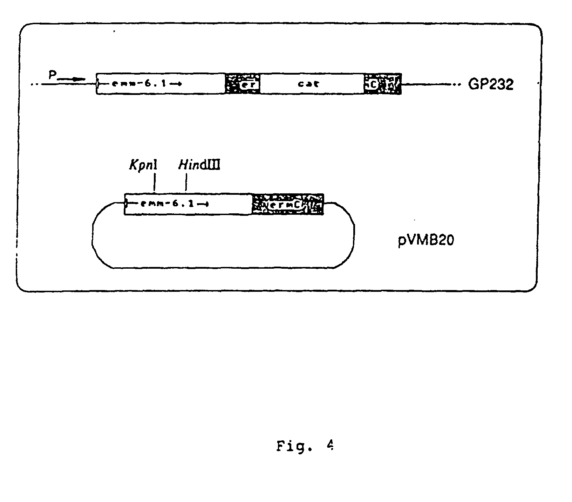

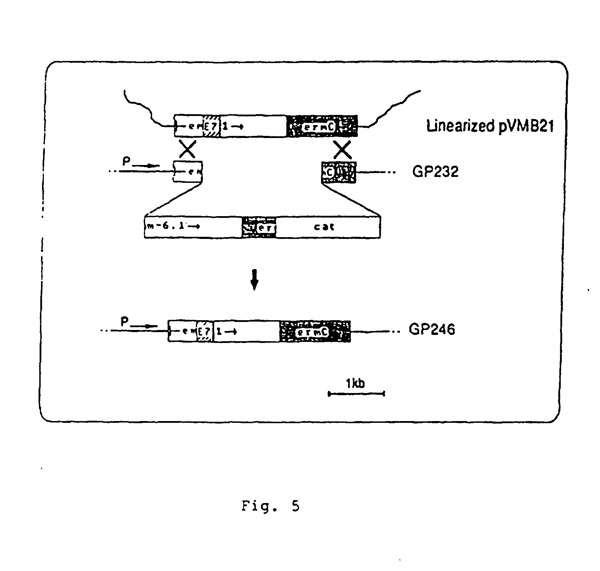

The novel bacteria of this invention may be produced by standard homologous recombination shown in Figs. 4 and 5. As illustrated, a new strain of Streptococcus gordonii, GP232 which expresses large quantitites of M6 protein was employed as the host to produce a new strain GP246.

The procedure employed as illustrated in Figs. 4 and 5 is standard and widely utilized by the skilled practitioner. The technology, as illustrated, is to construct a gram positive recipient organism for the insertion of the fusion gene in a random location in the chromosome that results in a high expression of the fusion gene. The insertion site may vary from organism to organism and even within the same organism. The advantage of the method is that it insures that the inserted gene is in a site which will result in high expression of the fusion gene, rather than rely on the expression as a result of a foreign promoter. Thus, the method assures that a region with a strong promoter is selected.

In the process of producing the novel GP246 strain, the 538 base pair segment between sites KpnI and HindIII of the M6 protein was excised from the novel plasmid pVMB20, produced as described below, and the 294 base pair encoding for E7 was inserted in its place to produce the novel gene which was utilized for the production of the new plasmid pVMB21. The region of the M protein that was excised results in the placement of the E7 molecule at the cell surface with a 120 amino acid segment of the M6 N-terminus including the leader segment fused to the N-terminus of E7. The construct was made in E. coli and the recombinant plasmid was used to transform S. gordonii by the standard double cross-over procedure with GP232 to effect chromosomal integration of the gene which expresses the M6:E7 protein by the new strain GP246.

### MATERIALS AND METHODS

**Recombinant DNA techniques.** Gene fusions in Escherichia coli vectors were obtained and constructed by standard procedures (30).

**Streptococcal transformation.** Frozen cells of naturally competent S. gordonii Challis were prepared and transformed by known procedures (31). Plating and scoring of transformants on multilayered plates was performed using erythromycin at a concentration 5 ug/ml in the overlay using known procedures (32).

**Genetic analysis of transformants.** Transformants were streaked on the surface of 3 blood agar plates by toothpick transfer of colonies from the selection plate. The first plate contained erythromycin (5 ug/ml), the second chloramphenicol (5 ug/ml), and the third had no antibiotic. Plates were incubated 36 hours at 37°C. After incubation, a nitrocellulose membrane was applied to the surface of the plate that contained no antibiotic, and kept for 20 min. at room temperature. The membrane was then incubated 30 min at 37°C and 15 min at 80°C in a vacuum oven. The presence of E7 protein bound to the nitrocellulose was detected using anti-E7 polyclonal antibodies (1:5000 dilution), obtained from rabbits immunized with a MS2:E7 fusion protein produced in E. coli (33).

**Immunofluorescence.** Bacteria grown in Todd-Hewitt broth (Difco) were harvested in late exponential phase, applied on a glass slide, and fixed with methanol. Slides were treated with M6- or E7-specific antibodies (1:50 dilution), extensively washed, and then reacted with goat anti-rabbit (or anti-mouse) IgG antiserum (1:100 dilution), conjugated with rhodamine. Results were observed under a Confocal Fluorescence Imaging System MRC-500 Bio-Rad microscope.

**Western blot analysis of cell extracts.** Streptococci were grown to late stationary phase in Todd-Hewitt berth (Difco). Cells were harvested and resuspended in 50 mM Tris(pH 8.0), 50 mM MgCl₂, 30% sucrose. Protoplasts were obtained by treating the cell suspension with lysozyme (100 ug/ml) for 30 min. at 0°C. Protoplasts were then centrifuged and resuspended in 50 mM Tris (pH 8.0). Thorough lysis was achieved by five cycles of quick freezing/thawing of the suspension. Cells that did not lyse and gross debris were discarded by low speed centrifugation at 1,000 rpm for 15 min, whereas the supernatant, containing membranes and cytoplasm, was used for Western blot analysis. The extract obtained from about 5X10⁸ streptococcal cells was run on a gel, and Western blot was performed by conventional methods.

**Immunization of mice.** BALB/c mice were immunized subcutaneously with 5X10⁸ live GP246 streptococcal cells (5X10⁷ colony forming units) emulsified in complete Freund's adjuvant. Two and three weeks after the primary immunization, animals were given subcutaneous boosters of the same bacterial dose emulsified in incomplete Freund's adjuvant. Animals were bled a week after the last boost.

### RESULTS

**Production of the plasmid pVMB3:** A construct was made where ermC, a gene conferring resistance to erythromycin (34), was cloned adjacent to emm-6.1, so that both genes would be within the same ClaI fragment. In this construct the initiation codon of emm-6.1 was 19 bp downstream of one of the ClaI sites, so that ClaI cleavage would leave emm-6.1 promoterless. Plasmid pVV3:M6 (35), containing this ClaI site upstream of the emm-6.1 coding sequence, was used in the experiments. The 2.0 kb MspI fragment of pE194, containing ermC, was ligated with a partial ClaI digestion of pVV3:M6. After transformation of E. coli DH5, a clone was isolated with a plasmid, pVMB3, containing the ClaI fragment.

**Production of the strain GP230:** The 3.4-kb ClaI fragment of pVMB3, containing ermC and the promoterless emm6.1, was ligated with chromosomal DNA of S. gordonii also cut with ClaI. The ligation mixture was used to transform the naturally transformable S. gordonii "Challis", strain V288. By this method the emm-6.1/ermC ClaI fragment was integrated at random into the chromosome. The chromosomal DNA ligated to the ClaI fragment provided the homology for integration during transformation. Erythromycin-resistant (Em-r) transformants were selected and analyzed for production of M6 protein by "streak blot". Of 700 Em-r transformants, 196 (28%) produced M6 protein. Based on the semiquantitative dot blot analysis, GP230 appeared to be the best M6 producer.

**Production of the strain GP232:** S. gordonii GP230 was transformed with the chromosomal DNA of pneumonococcal strain GP69. This pneumonoccal strain is resistant to chloramphenicol but susceptible to erythromycin produced according to the procedure of Pozzi and Guild (36). When GP69 chromosomal DNA is used to transform GP230, recombination occurs at the level of the ermC sequence leading to insertion of the CAT sequence into the copy of ermC integrated into the GP230 chromosome. This insertion yields GP232 which, as discussed and shown in the figures, expresses M6 on its surface and is also resistant to chloramphenicol and sensitive to erythromycin.

**Expression of E7 protein of HPV16 in S. gordonii:** The integration vector, pVMB20, was constructed to allow insertion of heterologous DNA sequences into the emm-6.1 gene present on the chromosome of GP232. pVMB20 is a novel Escherichia coli plasmid that does not replicate in Streptococcus and carries emm-6.1 and the erythromycin resistance marker ermC. The host-vector system GP232-pVMB20 is shown in detail in Figs. 4 and 5. Specifically, the novel plasmid pVMB20 and novel bacteria S. gordonii GP246 were prepared by the following procedure.

**Host-vector system for heterologous gene expression:** [A] In the chromosome of the novel host strain S. gordonii GP232, a copy of the M6 protein gene (emm-6.1)(37), promoterless but with its own ribosomal binding site was integrated downstream of a strong chromosomal promoter. Adjacent to emm-6.1 is found ermC (34), whose coding sequence is interrupted by insertion in its BclI site of the 1.8-kb MboI fragment of pC221 containing a cat gene (38). GP232 expresses M6 on its surface and is resistant to chloramphenicol and sensitive to erythromycin. It was obtained using transformation to integrate heterologous DNA into the streptococcal chromosome. The structure is shown in the figure. The size (5.2-kb) of the heterologous DNA integrated into the chromosome in GP232 was determined by Southern blot analysis. The integration vector pVMB20, is a 6.3-kb E. coli plasmid that does not replicate in Streptococcus. It was obtained by subcloning in pBLUESCRIPT (Stratagene, La Jolla, California) a 3.4-kb ClaI fragment of plasmid pVMB3 containing emm-6.1 and ermC by the procedure explained below. This is the same ClaI fragment which is integrated into the chromosome of GP232, the only difference being that in GP232 ermC is interrupted by cat. When pVMB20 is used as donor DNA in transformation of competent cells of S. gordonii GP232, erythromycin-resistant transformants are obtained by recombination between the integration vector and the homologous chromosomal sequences. The DNA fragment containing the cat gene is deleted in the chromosome of these transformants, whereas an intact ermC gene is restored. (Fig. 5)

(B) The E7 protein gene of HPV16(39) was cloned into the emm-6.1 sequence of pVMB20 to yield pVMB21. pVMB20 was digested with KpnI and HindIII and ligated with a KpnI/HindIII segment containing the E7 sequences obtained by in vitro DNA amplification (polymerase chain reaction) performed on plasmid pMBS21L/E7 (33). Amplification primers were designed in order to obtain "in frame" insertion of the 294 bp encoding for E7 into emm-6.1. Nucleotide sequence analysis of pVMB21 confirmed the expected structure of the M6:E7 translational fusion. pVMB21 was linearized and used to transform GP232. E7 was found to be expressed in 6% of the erythromycin-resistant transformants. In these transformants integration of the pVMB21 sequences produced a deletion involving the cat gene. The structure of GP246, a representative transformant, was confirmed by Southern blot analysis. The nucleotide sequence of the junction fragments of the M6:E7 gene fusion present on the chromosome of GP246 was also determined after cloning in pBLUESCRIPT the ClaI fragment containing the M6:E7 fusion.

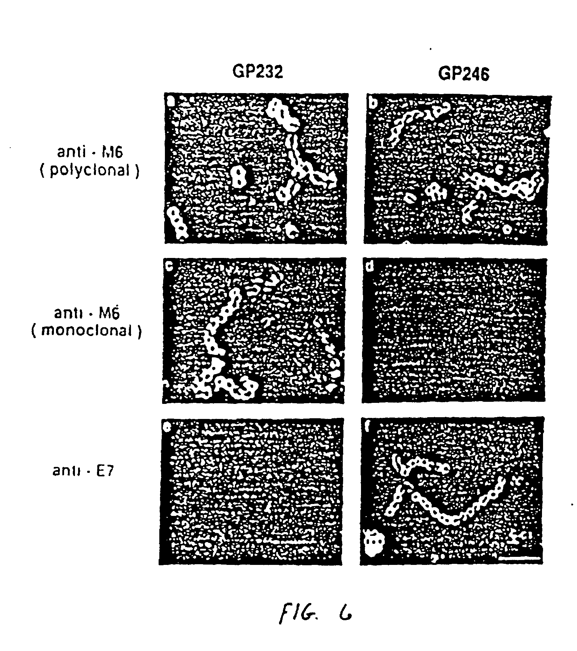

**Surface expression of the E7 protein:** Expression of the E7 protein of HPV16 in S. gordonii on the surface of strain GP246 was verified by immunofluorescence using antibodies specific for either the M6 protein carrier or the E7 insert. GP246, containing the M6:E7 gene fusion exhibited positive fluorescence when reacted with either M6-specific or E7-specific polyclonal antibodies (Fig. 6), confirming the surface location of the E7 molecule and the M protein on the surface of S. gordonii. No fluorescence was observed when GP246 was reacted with known monoclonal antibody 10A11, which is specific for an epitope of M6 whose coding region was contained in the KpnI/HindIII fragment deleted in the construction of M6:E7 gene fusion (Fig. 6).

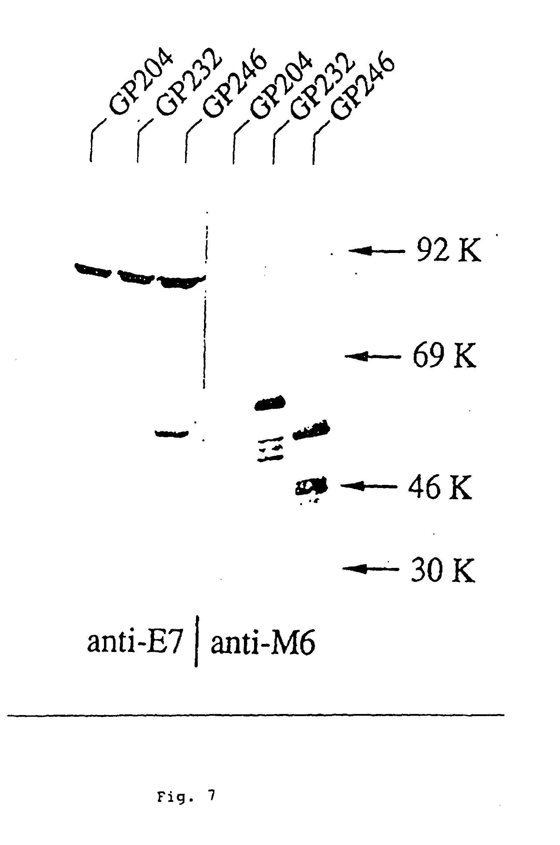

To demonstrate that the E7-expressing recombinant streptococci in fact produce an M6:E7 fusion protein, S. gordonii cell extracts were analyzed by Western blot (Fig. 7). In cell extracts of GP246, the same bands reacted with E7- and M6-specific antibodies, whereas no E7-specific reactivity was found in the recipient GP232, whose extracts showed M6-specific reactivity (Fig. 7).

Substantially the same procedures used to construct M6:E7 a hybrid protein containing the C-terminal region of the M6 molecule and allergen 5 was successfully expressed on the surface of S. gordonii. Western blots of the cell wall extract using allergen 5 specific antibodies established (as with M6:E7) that allergen 5 was indeed expressed in the cell wall fraction of the gordonii. Allergen 5 was obtained as described by Fang, et al (66).

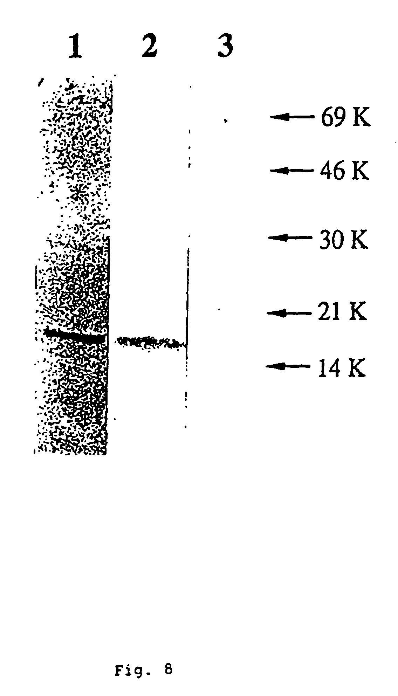

**Immune response to fusion protein on streptococcal surface:** The immunogenicity of the M6:E7 fusion protein was examined by immunizing mice with recombinant S. gordonii GP246 expressing the M6:E7 fusion protein. Control mice were immunized with the isogenic strain GP232 which express M6 protein. Sera from three animals immunized with each strain were pooled and tested by Western blot for their reactivity with purified E7 protein produced in Schizosaccharomyces pombe. Fig. 8 shows that animals immunized with strain GP246 containing surface M6:E7 produced antibodies reactive with the E7 protein, indicating that E7 protein is immunogenic when expressed as a fusion protein on the streptococcal surface. No antibodies to the E7 protein were seen in the sera of mice immunized with GP232 containing only M6.

The procedure which has been described permits the production of the non-pathogenic bacteria S. gordonii GP246 or other transformed gram positive bacteria which express a hybrid surface protein such as the novel hybrid surface antigen M6:E7. This novel antigen is a hybrid protein, the carboxy terminus of which is attached to the bacteria and is substantially the same C-terminal region normally found in other gram-positive surface proteins as explained above. The active polypeptide of this hybrid surface protein is the antigen E7 of HPV 16. When the bacteria is administered, e.g. by colonization, to an animal host in need of protection, the active polypeptide will elicit both humoral and cell mediated responses resulting in the production of a protective immune response against infection by papillomavirus.

It will be noted that the heterologous antigen of the hybrid surface protein of this invention prepared as described above is a viral antigen produced during viral replication. Thus, the process of this invention makes possible the production of novel gram positive bacteria for the delivery of viral antigens to an animal in sufficient quantities to elicit a protective immunogenic response to infection by a virus.

The novel M6:E7 and novel intermediate products of this invention are produced from two starting materials. These are pVV3:M6 and GP69. They can be prepared by the procedures described in the cited references. Therefore the novel products can all be obtained from known materials utilizing the procedures described herein. However, to assist in the practice of the invention and without admitting any necessity to do so pW3:M6 and GP69 have been deposited at the American Type Culture Collection under the accession numbers ATCC 68003 and ATCC , respectively.

The foregoing M6:E7 example illustrates the utilization of a non-pathogenic commensal gram positive bacteria normally present in the mammalian oral cavity to protect the whole body against viral infection. Analogously prepared non-pathogenic bacteria can be used to express and deliver other useful antigens by the processes described and illustrated herein. For example, protein antigens on the surface of mammalian tumors can be fused with the cell wall associated region of a surface antigen of any gram positive bacteria, the fusion gene inserted in a gram positive commensal, and the resulting bacteria employed as a vaccine to generate tumor specific immune responses useful in tumor therapy.

These examples illustrate only one aspect of the invention. The invention, in fact, provides a delivery system for any kind of polypeptide which may be useful to raise an immune response in an animal or for other useful purposes.

When utilized as a vaccine, the selected transformed commensal will be employed to colonize a mammal. It will produce the selected hybrid surface protein, the active polypeptide segment of which will elicit the production of protective antibodies.

Gram positive non-pathogenic bacteria normally found on the vaginal mucosa may be employed as contraceptives. For this utility, surface antigens of male sperm can be employed as the active polypeptide on the hybrid surface protein of the bacteria Lactobacillus acidophilus normally found on the surface of the vaginal or uterine mucosa. When the transformed bacteria are utilized to colonize such mucosa, the hybrid proteins will elicit the production of antibodies to the surface antigens of the sperm and bring about inactivation of the sperm. In effect, the sperm will be viewed by the female body as a foreign substance and the preformed antibodies will react with and inactivate the sperm cells.

There are two important advantages to this type of contraception. One is that, since the sperm will be inactivated, the ova will not be fertilized. The other is that the contraceptive effect can be neutralized simply by treating the immunized female with an antibiotic to clear the gram positive bacteria expressing the hybrid antigen.

Still another utility of the transformed bacteria of this invention is the delivery of a surface antigen from an HIV virus to the surface of a gram-positive commensal organism to provide a vaccine which may be used to prevent AIDS. For this utility, the polypeptide GP120, the V3 loop of GP120, GP160 or a related surface antigen or segments of such antigens such as sequence 735 to 752 of GP16 or RP135 a 24-amino acid segment of GP 120 from the HIV virus. These polypeptides will be fused to the leader and at least a portion of the N-terminal sequence of a gram positive surface protein and its anchor region for delivery to the surface of the commensal bacteria. When the transformed commensal expressing the HIV antigens on its surface is delivered to a susceptible mammal, an immune response is raised to protect against infection by HIV virus. Furthermore, by using a recombinant vaginal commensal to deliver an HIV antigen such as GP120 to the vagina, the IgA antibodies produced to this antigen are protective against infection at this site. IgA antibodies in vaginal secretions will reduce the number of HIV particles in the secretions of an HIV positive female and thus reduce the spread of AIDS.

The products of this invention are useful in desensitization therapy. This type of therapy is widely employed to alleviate the discomfort of atopic individuals who develop exaggerated IgE responses to antigens (called allergens in the allergy field). The increased production of IgE antibodies is believed to be a principal cause of the allergic responses such as hay fever, asthma, and anaphylactic shock.

The allergens may arise from any of a variety of sources including foods, molds, dust and animal furs. Flora such as roses or rag weed are a major source of allergens. The "sting" of vespids such as wasps, yellow jackets and hornets contain allergenic proteins.

The conventional therapy employed to ameliorate the immune response from exposure to allergens has been hyposensitization treatment which involves repeated injections of increased doses of allergen. This causes an increase in allergen specific IgG antibodies which blocks the binding of allergen specific IgE to mast cells.

A number of allergen-specific peptides and proteins have been identified, isolated, and cloned. Those include, for example, allergen 5, a vespid venom protein. These allergens can be included as the active polypeptide of the hybrid surface proteins of this invention. Commensal bacteria which generate such hybrid proteins may be used to colonize an allergic patient. The result will be a constant source of allergens which will elicit the same protective immune response as achieved with desensitization therapy.

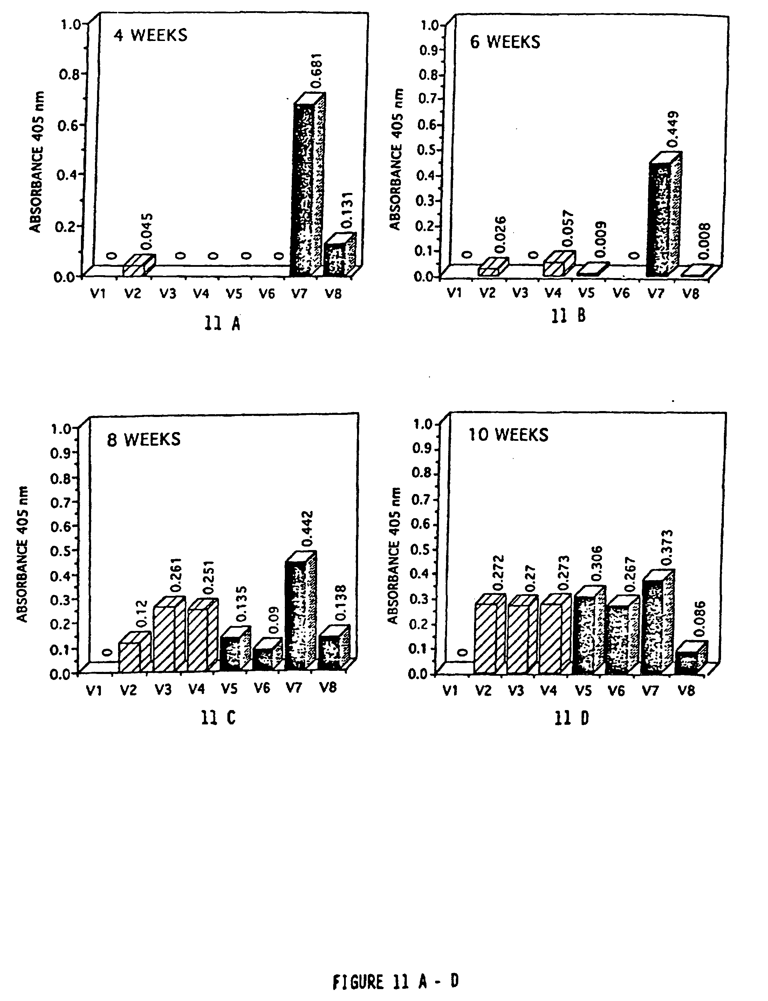

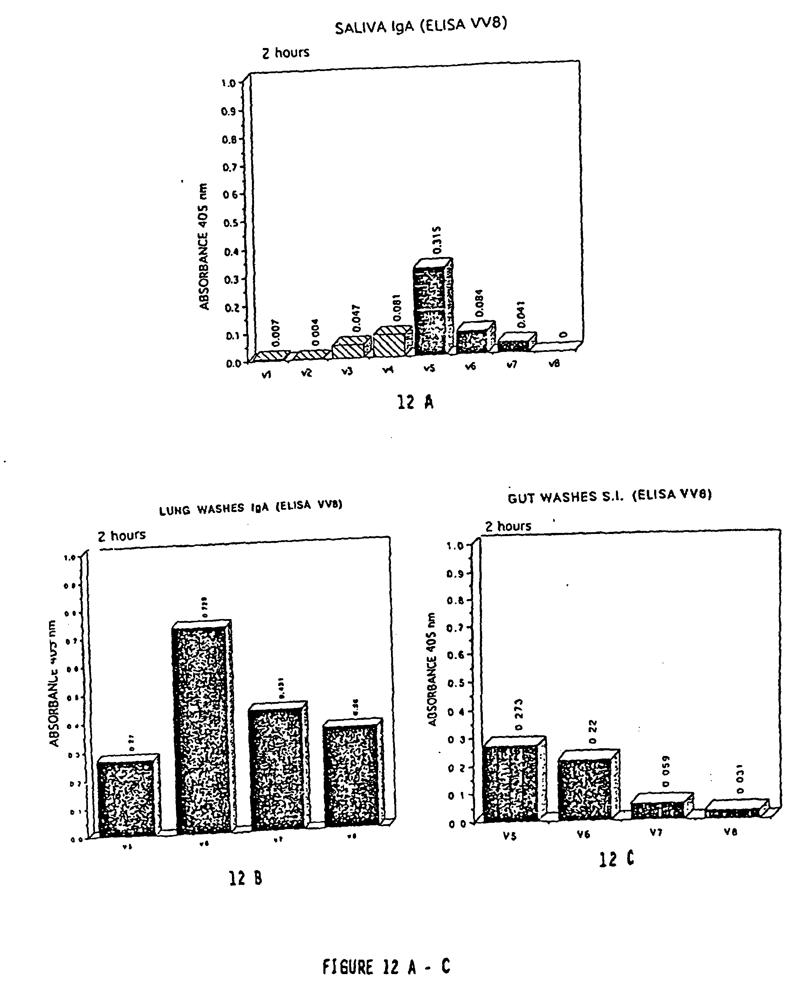

The formation and expression of the hybrid protein M6:allergen 5 has been described above. Figs 11 and 12 show the results of immunization studies utilizing this hybrid surface antigen.

Fig. 11 illustrates the serum IgG response of mouse sera to purified allergen 5 in the hybrid surface protein. Mice were immunized either intradermally with S. gordonii expressing hybrid surface allergen 5 (10⁷ CFU) in Freund's adjuvant or intranasally with the same organisms. Animals were bled at 4, 6, 8, and 10 weeks after immunization and the serum checked by ELISA for IgG to purified allergen 5. Both mice immunized intradermally (V1-V4, hatched bars) and animals immunized intranasally (V5-V8, closed bars) expressed antibodies to the allergen. Control animals immunized with S. gordonii without surface exposed allergen-5 showed no response to the allergen. All animals immunized intranasally with the recombinant S. gordonii remained colonized for at least 12 weeks as determined by swabs and still expressed the allergen 5 for this period. All assays were performed with a 1:50 dilution of sera.

Fig. 12 shows the IgA response to allergen 5 in intranasally and intradermally immunized animals. Twelve weeks after immunization (intradermally or intranasally) with gordonii expressing allergin-5 (see Fig. 11) saliva was taken and the animals sacrificed to obtain lung and gut washes. The saliva and washes were analyzed for the presence of IgA specific for allergen-5 by ELISA. Results revealed that salivary IgA was present in the intradermal and intranasally immunized animals with a better response in the intranasally colonized animals. The best IgA response was seen in the lung washes of the intranasally colonized animals. While an IgA response was seen in the small intestine of the gut (S.I.), they were lower than the lung washes. All assays were performed with a 1:1 dilution of the samples.

A particular advantage of the systems for delivery of the hybrid surface proteins of this invention is that since the transformed organisms are normal commensals, they will continue to colonize and act as a constant stimulus of a protective immune response thereby avoiding the need of continual immunization.

The skilled artisan can readily conceive of numerous other applications of the novel products of this invention. For example, non-pathogenic gram positive bacteria can be produced to deliver surface antigens which will elicit protective antibodies against a wide variety of disease states including malaria, measles, pertussis, retroviral infections such as AIDS, coccidiosis, distemper, fowl pox and brucellosis.

Another particular advantage of the delivery system of this invention when used as vaccine is that live bacteria are employed. As is known, live bacteria stimulate a much stronger immune response than attenuated or dead bacteria. Still another advantage is that, because non-pathogenic bacteria are employed, the vaccine is safe. Still another is that several methods of administration are possible.

The invention is not limited to S. gordonii as the carrier bacteria or even to streptococcus, in fact other non-pathogenic gram-positive bacteria may often be preferred. For example, bacteria normally present on the intestinal mucosa (Enterococcus fecalis) may be preferred to deliver an active polypeptide to the intestine.

Moreover, the invention is not limited to the surface expression of one hybrid surface protein containing only one epitope. The carrier organism may be transformed by well known procedures to express a plurality of hybrid surface proteins each with a different active polypeptide or, alternatively, to express a hybrid surface protein in which the active polypeptide includes more than one epitope.

Thus far this invention has been described and illustrated principally with respect to the delivery of antigens to elicit protective antibodies but, as indicated above, the invention is not so limited. For example the invention can be used to deliver enzymes.

Humans deficient in the enzyme lactase which is secreted in the small intestine are unable to properly hydrolyze lactose into its components, D-glucose and D-galactose. One result is that they are unable to digest milk or milk products properly. Heretofore, the treatment has been life long ingestion of tablets or other therapeutic dosage forms containing the missing enzyme. In accordance with the practice of this invention, commensal intestinal bacteria are provided which are cloned to produce a hybrid protein, the active polypeptide of which is lactase. The bacteria may be employed to colonize the intestinal mucosa for the continual production of the required enzyme.

Streptococcus mutans are major organisms responsible for tooth decay. They secrete glucosyltransferases which hydrolyze sucrose to produce glucose. The glucose, in turn, is formed into glucan polymers which adhere to the tooth surfaces and participate in the decay process. Utilizing the procedures described and claimed herein, S. mutans can be transformed to express hybrid surface proteins containing glucanase enzymes. The transformed bacteria may be employed to colonize the oral cavity and inhibit the formation of glucan polymers thereby protecting against tooth decay. Alternatively, Streptococcus mutans may be transformed to produce enzymes that digest dental plaque, thus preventing the initiation of decay.

The process of this invention substantially as described above was employed utilizing Staphyloccus aureus as the bacteria for expression of the surface protein. When the anchor region (containing the LPXTGX segment, hydrophobic segment and charged tail segment) of Protein A, a surface molecule of this bacteria was fused to the enzyme alkaline phosphatase (a dimeric periplasmic protein of E. Coli) and the gene for this fusion placed back in S. aureus, the molecule was found to be translocated to the outside of the cell and anchored to its surface. The details of this procedured are found in Reference 65.

The skilled artisan will recognize that several variations of this invention are possible.

One variation which is particularly useful, especially for the production of vaccines is to include an adjuvant in the hybrid protein and the necessary nucleotide sequence for the expression of the desired protein in the gene used to generate the protein. Typically, a plasmid could be produced in which the gene for expressing the cholera toxin B subunit which is a known mucosal adjuvant is inserted between the C-region polypeptide and the active polypeptide. Alternatively, the gene for the subunit could be inserted upstream of the active polypeptide. This orientation may elicit an enhanced IgA response because in the usual interaction of CTB and the cell which produces antibodies, the CTB directly contacts the cell and stimulates the production of antibodies for the antigen for which CTB is functioning as an adjuvant. The plasmid may be employed in the double cross-over technique illustrated in Figs. 4 and 5 to incorporate the hybrid gene into the chromosome of a gram positive bacteria for the expression of a hybrid protein of the invention on the bacterial surface. Other immune enhancing proteins may be used instead of the cholera toxin B subunit to enhance the immune response to an active polypeptide.

The transformed bacteria of this invention are useful in diagnostic tests. They may be used, for example, to test for the presence of antibodies characteristic of a specific infection in the plasma of infected animals.

Conventionally, the procedure of testing for an infection caused by, for example, Neisseria gonorrhoeae requires the isolation and purification of an antigen which will react with a specific antibody characteristic of the infecting microorgansim and suspected to be present in the plasma. The antigen is then incubated with plasma or other body fluid. If a reaction takes place, it can be recognized by any of a variety of known tests.

In the standard enzyme linked immunoassay procedure, the isolated and purified antigen is adhered to a solid phase glass or plastic substrate. It is then exposed to the body fluid such as plasma or serum. If the fluid contains an antibody characteristic of the infecting organism, an antigen/antibody reaction will take place. The reaction product will remain on the solid phase which is then incubated with an anti-antibody tagged with a detectable enzyme such as horseradish peroxidase.

The transformed bacteria of this invention can be employed as the source of the antigen used in the diagnostic test. In such tests, the transformed bacteria is employed as an antigen. The skilled artisan will be familiar with several diagnostic tests of this nature. Such use, in accordance with this invention, eliminates the necessity of isolating and purifying the antigen as required by prior art methods.

For diagnostic testing, a transformed bacteria of the invention is prepared in which the active polypeptide includes an epitope for a specific antibody which is characteristic of the microorganism causing the infection. The bacteria is adhered to the solid phase and the balance of the diagnostic test is conducted in the usual manner. Of course the test is not limited to the use of enzymes to detect the presence of antibody utilizing, for example, the ELISA assay. Other labels such a radioactive isotopes can be employed.

Diagnostic kits can be provided containing the transformed bacteria, typically in a lyophilized form to be reconstituted, together with the other reagents normally provided with such kits. These may include an aqueous buffer, a labeled anti-antibody, sterilized water and, possibly, other reagents. Alternatively, the transformed bacteria, either in aqueous suspension of lyophilized may be provided for use in the diagnostic test together with other reagents to be provided by the laboratory conducting the diagnostic test.

The various segments of the hybrid surface proteins of the invention are not necessarily adjacent to each other in the protein or in the gene used to produce it. It may be convenient for any of a number of reasons apparent to the skilled artisan to separate the nucleotides on the hybrid gene used to express the protein by insertion of other nucleotides thereby to express hybrid proteins in which the active polypeptide and the cell associated polypeptide are separated by a selected spacer. It is not essential that the complete segment from the cell associated region of the surface antigen be employed in constructing the hybrid surface protein of the invention so long as an anchor region beginning with the LPXTGX segment and ending at the charged tail segment is present to anchor the hybrid polypeptide to the bacteria that expresses it.

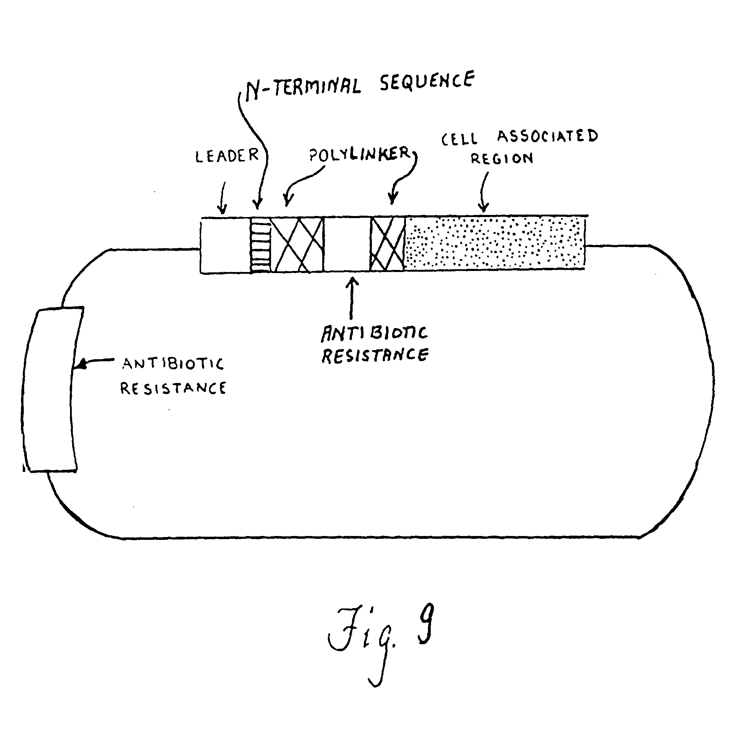

Fig. 9 shows one especially useful embodiment of this invention. It illustrates a universal plasmid containing a polylinker site. A polylinker is an oligonucleotide or DNA sequence which contains several restriction sites and therefore can be employed for the insertion of genes excised by a variety of restriction enzymes. For example, the plasmid can be constructed with a polylinker insert containing several restriction sites such as EcoRI, ClaI, HindIII, XboI, PstI, BamHI or any of several others. Such polylinkers are either commercially available or may be readily constructed by the artisan.

To produce a plasmid of this invention the polylinker insert will be ligated into a plasmid already containing the selected gene segment for expressing the desired cell associated segment of the hybrid protein to be produced. The plasmid is shown as containing the leader sequence for the hybrid antigen, but such sequence is not necessarily in the plasmid. It may be included in the oligonucleotide sequence used to express the active polypeptide. Although the various gene segments in the plasmid are shown as adjacent to each other, they may be separated by spacers.

The plasmid is shown as containing two different antibiotic resistant regions to assist in selection. One, which is located within the polylinker site, separates it into two sections and will be removed when replaced by an insert. Other markers such as those resulting in color may be employed. Other formulations of a plasmid with polylinker sites may be constructed as required.

The plasmid without the marker which separates the linker, but with an effective promoter, can be employed to transform a gram positive bacteria. The plasmid shown can cross over with a chromosome as described above. A transformed bacteria containing the plasmid or the chromosome will express the desired surface hybrid protein.

The plasmid M6.1 367-441 which is described and claimed in the above identified parent application is especially useful in the practice of this invention. The preparation of this plasmid is described in the parent application. It has been deposited in E. coli strain K561 at the American Type Culture Collection under the accession number ATCC68235.

As explained in the parent applications, this plasmid can be fused with an appropriate DNA sequence and the hybrid gene inserted in an appropriate carrier, such as E. coli or a selected non-pathogenic gram positive bacteria. The resulting transformed bacteria will express a polypeptide of this invention. The transformed gram positive bacteria may be used directly as a vaccine. Alternatively, it can be used in a cross-over reaction as explained above to produce another transformed bacteria which will express the desired polypeptide more efficiently. The plasmid may also be used for the production of a unviersal peptide by the insertion of a polylinker.

A special advantage of the invention is that the recombinant gram positive bacteria may be delivered to the animal in need at the mucosal site at which the pathogen normally invades the body. These include oral as well as nasal, intestinal and vaginal sites. Such delivery will stimulate a local immune response at the site and be an effective means to raise a protective immune response to the pathogen. Since the bacteria employed will be non-pathogenic, normal organisms for that particular site, they may be employed without danger of toxic effects. The selected bacteria can be administered to fish or wild animals by mixing it in the food they eat or the water they inhabit.

Utilizing the same procedures described above, hybrid proteins can be prepared containing the antibody eliciting antigens shown in the following Table 2 as active polypeptides. The table shows the polypeptide, which will produce antibodies protective against infection by a pathogen, the disease to be protected against and the publication which describes the polypeptide. In some instances the gene used to elicit the oligonucleotide coding for a peptide will need to be prepared and inserted in a plasmid. The procedures are completely parallel to the procedures described above. The C-terminal anchor region can be any of those specifically suggested above, or any other anchoring segment of a surface protein from a gram-positive bacteria described in Table 1. The active polypeptide of the transformed bacteria will include the peptide shown, fused to the leader sequence and a small segment of the N-terminus of a surface protein to permit proper translocation to the cell surface.

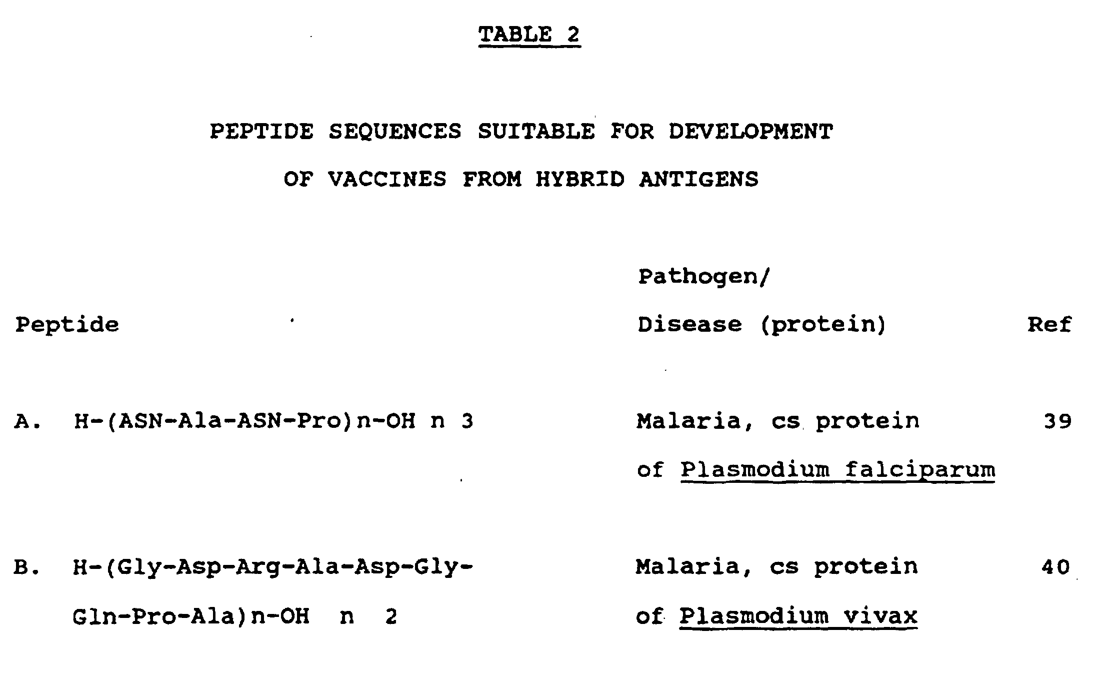

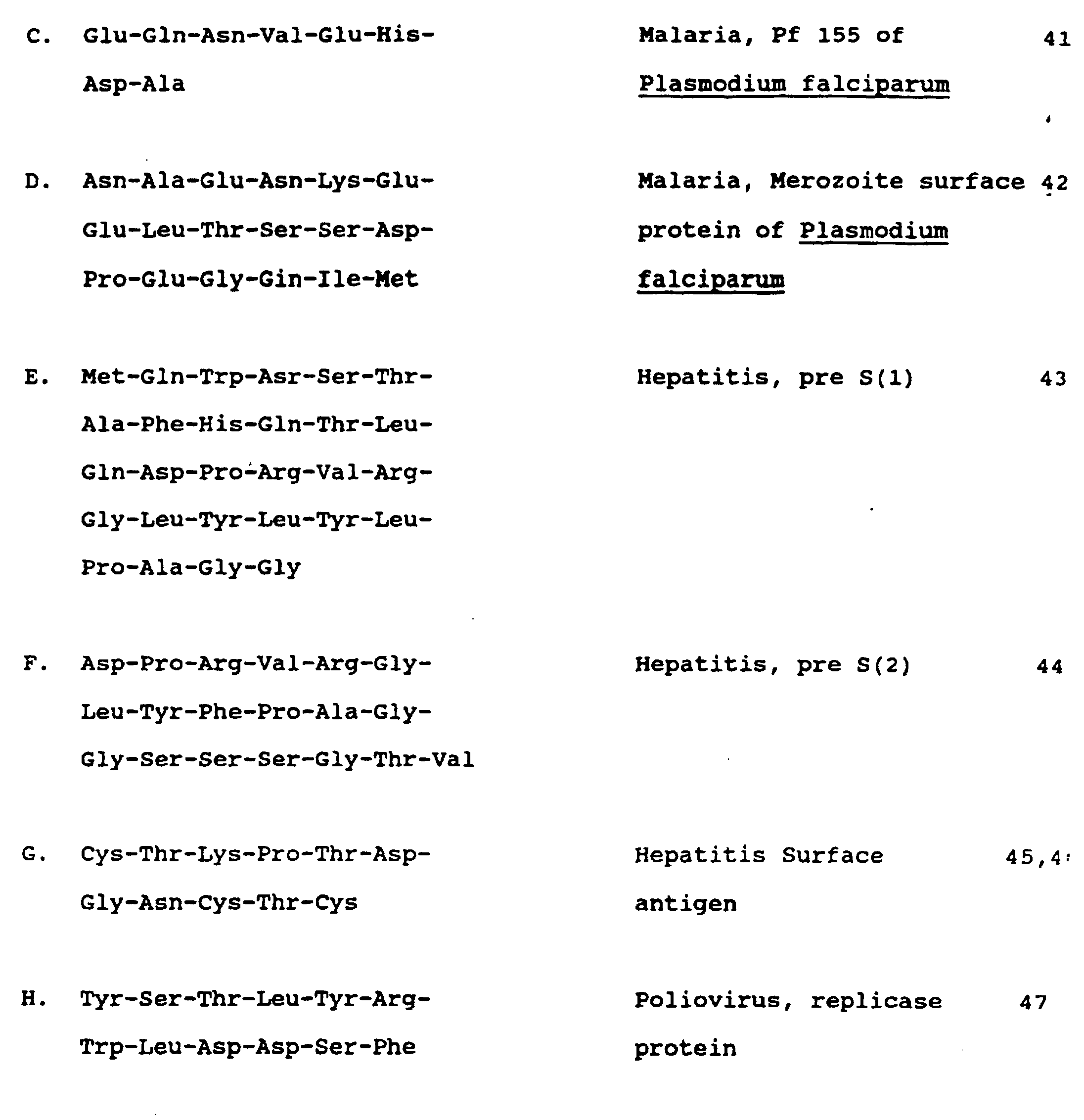

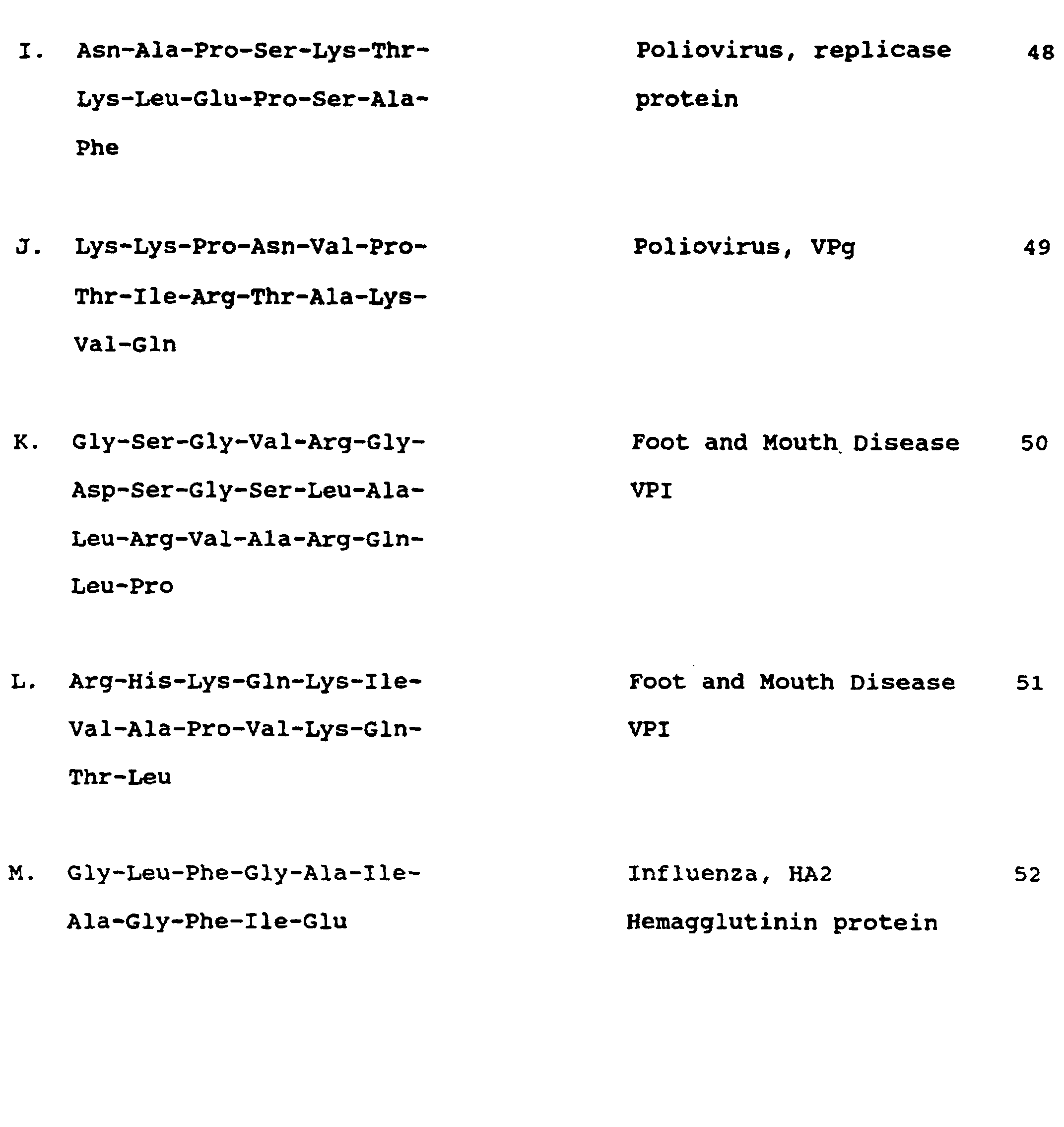

The transformed bacteria of the invention are, in effect, therapeutic agents, and will be treated as such. They may be used alone or in association with pharmaceutically acceptable carriers of the type usually employed with such therapeutic agents. For oral or nasal administration, they may be suspended in aqueous isotonic saline which may be flavored or colored. For intestinal delivery, they may be placed in a capsule to deliver the transformed bacteria orally. Other methods of administration will be readily apparent to the skilled artisan and are within the scope of the invention.

Since the products of the invention are utilized as live transformed bacteria to colonize the selected site of administration, no specific dosage is required. Typically, the selected dosage form will contain from about 10⁶ to 10¹⁰ organisms per dosage unit.

### REFERENCES

1. Fischetti. V.A., V. Pancholi and O. Schneewind. (1990) Mol. Microbiol. 4:1603.
2. Hollingshead, S.K., V.A. Fischetti, and J.R. Scott. 1986. J. Biol. Chem. 261:1677.
3. Miller, L., L. Gray, E.H. Beachey, and M.A. Kehoe. 1988 J. Biol. Chem. 263:5668.
4. Robbins, J.C., J. G. Spanier, S.J. Jones, W.J. Simpson, and P.P. Cleary. 1987. J. Bacteriol. 169:5633.
5. Mouw, A.R., E.H. Beachey and V. Burdett, 1988. J. Bacteriol. 170:676.
6. Haanes, E.J. and P.P. Cleary. 1989. J. Bacteriol. 171:6397.
7. Manjula, B.N., K.M. Khandke, T. Fairwell, W.A. Relf, and K.S. Sripakash. 1991. J. Protein Chem. 10:369.
8. Bessen, D.E. and V.A. Fischetti. 1992. Infect. Immun. 60:124.
9. Frithz, E., L-O. Heden, and G. Lindahl. 1989. Molec. Microbiol. 3:111.
10. Heath, D.G. and P.P. Cleary. 1989. Proc. Natl. Acad. Sci. U.S.A. 86:4741.
11. Gomi, H., T. Hozumi, S. Hattori, C. Tagawa, F. Kishimoto, and L. Bjorck. 1990. J. Immunol. 144:4046.
12. Chen, C.C. and P.P. Cleary. 1990. J. Biol. Chem. 265:3161.
13. Schneewind, O., K.F. Jones and V.A. Fischetti. 1990. J. Bacteriol. 172:3310.
14. Heden, L-O, E. Frithz, and G. Lindahl. 1991. Eur. J. Immunol. 21:1481.
15. Olsson, A., M. Eliasson, B. Guss, B. Nilsson, U.. Hellman, M. Lindberg, and M. Uhlen. 1987. Eur. J. Biochem. 168:319.
16. Okahashi, N., C. Sasakawa, S. Yoshikawa, S. Hamada, and T. Koga. 1989. Molec. Microbiol. 3:673.
17. Kelly, C., P. Evans, L. Bergmeier, S.F. Lee, -Fox Progulske, A., A.C. Harris, A. Aitken, A.S. Bleiweis, and T. Lerner. 1990. FEBS Lett. 258:127.
18. Tokuda, M., N. Okahashi, I. Takahashi, M. Nakai. S. Nagaoka, M. Kawagoe, and T. Koga. 1991. Infec. Immun. 59:3309.
19. Ferretti, J.J., R.R. B. Russell, and M.L. Dao. 1989. Molec. Microbiol. 3:469.
20. Kao, S-M., S.B. Olmsted, A.S. Viksnins, J.C. Gallo, and G.M. Dunny. 1991. J. Bacteriol. 173:7650.
21. Galli, D., F. Lottspeich, and R. Wirth. 1990. Molec. Microbiol. 4:895.
22. Guss, B., M. Uhlen, B. Nilsson, M. Lindberg, J. Sjoquist, and J. Sjodahl. 1984. Eur. J. Biochem. 138:413.
23. Signas, C., G. Raucci, K. Jonsson, P. Lindgren, G.M. Anantharamaiah, M. Hook, and M. Lindberg. 1989. Proc. Natl. Acad. Sci. USA 86:699.
24. Kok, J., K.J. Leenhouts, A.J. Haandrikman, A.M. Ledeboer, and G. Venema. 1988. Appl. Environ. Microbiol. 54:231.
25. Gaillard, J.K., P. Berche, C. Frehel, E. Gouin, and P. Cossart. 1991. Cell 65:1.
26. Yeung, M.K. and J.O. Cisar. 1990. J. Bacteriol. 172:2462.
27. Yeung, M.K. and J.O. Cisar. 1988. J. Bacteriol. 170:3803.
28. Schneewind, O., V. Pancholi, and V.A. Fischetti. 1991. In: Genetics and Molecular Biology of Streptococci, Lactococci and Enterococci. G.M. Dunny, P.P. Cleary and L.L. McKay. ASM Publications, Washington. 152.
29. Pancholi, V. and V.A. Fischetti. 1988. J. Bacteriol. 170:2618-2624.
30. Maniatis, T., E.F. Fritsch and J. Sambrook. 1982. Molecular Cloning, Cold Spring Harbor Laboratory, Cold Spring Harbor.
31. Pozzi, G., R.A. Musmanno, P.M. Lievens, M.R. Oggioni, P. Plevani, and R. Manganelli. 1990. Res. Microbiol. 141:659-670.
32. Pozzi, G., R.A. Musmanno, M. Stellini, and A.M. Molina. 1987. FEMS Microbiol. Lett. 48:189.
33. Tommasino, M., M. Contorni, V. Scurlato, M. bugnoli, K. Maundell, and F. cavalieri. 1990. Gene 93:265.
34. Horinouchi, S. and B. Weisblum. 1982. J. Bacteriol. 150:804.
35. Hruby, D.E., W.M. Hodges, E.M. Wilson, C.A. Franke, and V.A. Fischetti. 1988. Proc. Natl. Acad. Scid. USA 85:5714.
36. Pozzi, G., W.R. Guild. 1985. J. Bacteriol. 161:909.
37. Hollingshead, S.K., V.A. Fischetti, and J.R. Scott. 1986. J. Biol. Chem. 261:1677-1686.
38. Wilson, C.R., S.E. Skinner, and W.V. Shaw. 1981. Plasmid 5:245-258.
39. Schwarz, E., U.K. Freese, L. Gissmann, W. Mayer, B. Roggenbuck, A. Stremlau, and H. zur Hausen. 1885. Nature 314:111-114.
40. Zavala, et al Science (1985) 228: 1436
41. McCutchan, et al Science (1985) 230: 1381
42. Udomsangpetch, et al Science (1986) 231: 57
43. Ravetch, et al Science (1984) 227: 1593
44. Neurath, et al Science (1984) 224: 392
45. Itoh, et al Proc. Natl. Acad. Sci. USA (1986) 83: 9174
46. Prince, et al Proc. Natl. Acad. Sci. USA (1982) 79: 579
47. Bhatnager, et al Proc. Natl. Acad. Sci. USA (1982) 79: 4400
48. Baron, et al Journal of Visology (1982) 43: 969
49. Baron, et al Cell (1982) 28: 395
50. Bittle, et al Nature (London) (1983) 298: 30
51. Atassi, et al Proc. Natl. Acad. Sci. USA (1982) 80: 840
52. Muller, et al Proc. Natl. Acad. Sci. USA (1982) 79: 569
53. Wang, et al Proc. Natl. Acad. Sci USA (1986) 83: 6159
54. O'Toole, P., L. Stenberg, M. Rissler, and G. Lindahl. 1992. Two major classes in the M protein family in group A streptococci. Proc. Natl. Acd. Sci. USA 89:8661.
55. Rakonjac, J. V., J.C. Robbins, and V.A. Fischetti. 1992. Cloning and sequencing of the gene encoding the serum opacity factor of Streptococcus pyogenes: variation among different M types Infect. Immun. 63:622.
56. Talay, R.S., P. Valentin-Weigand, P.G. Jerlstrom, K.N. Timmis, and G.S. Chhatwal. 1992. Fibronectin-binding, protein of Streptococcus pyogenes: sequence of the binding domain involved in adherence of streptococci to epithelial cells. Infect. Immun. 60:3837.
57. Kastern, W., U. Sjobring, and L. Bjorck. 1992. Structure of peptostreptococcal protein L and identification of a repeated immunoglobin light chain-binding domain J. Biol. Chem. 267:12820.
58. Michel, J.L., L.C. Madoff, K. Olson, D.E. Kling, D.L. Kasper, and F.M. Ausubel. 1992. Large, identical, tandem repeating units in the C protein alpha antigen gene, bca, of group B streptococci. Proc. Natl. Acad. Sci. USA 89:10060.
59. Lindgren, P-E., M.J. McGavin, C. Signas, B. Guss, S. Gurusiddappa, M. Hook, & M. Lindberg. 1992. Two different genes coding for fibronectin-binding proteins from Streptococcus dysgalactiae. The complete nucleotide sequences and characterization of the binding domains. Eur. J. Biochem. 214:819.
60. Collins, C.M., A. Kimura, and A.L. Bisno. 1992. Group G streptococcal M protein exhibits structural features analogous to those of class I M protein of group A streptococci. Infect. Immun. 60:3689.
61. Sjobring, u. 1992. Isolation and molecular characterization for a novel albumin-binding protein from group G streptococci. Infect. Immun. 60:3601.
62. Smith, H.E., U. Vecht, A.L.J. Gielkens, and M.A. Smits. 1992. Cloning and nucleotide sequence of the gene encoding the 136-kilodalton surface protein (muramidase-released protein) of Streptococcus suis type 2. Infect. Immun. 60:2361.
63. Jonsson, J., C. Signas, H-P. Muller, and M. Lindberg. 1991. Two different genes encode fibronectin binding proteins in Staphylococcus aureus. Eur. J. Biochem. 202:1041.
64. Patti, J.M., H. Jonsson, B. Guss, L.M. Switalski, K. Wiberg, M. Lindberg, and M. Hook. 1992. Molecular characterizations and expression of a gene encoding a Staphylococcus aureus collagen adhesin. J. Biol. Chem. 267:4766.
65. Schneewind, et al Cell (1992) 70:267
66. Fang, et al Proc. Natl. Acad. Sci. USA (1988) 85:895

## Claims

1. A hybrid surface protein including the anchor sequence LPXTGX of a surface antigen normally expressed by a gram positive bacteria fused to an active polypeptide which may be delivered to a mammalian host for a useful purpose.

2. A hybrid surface protein of claim 1 wherein the active polypeptide is an enzyme.

3. A hybrid surface protein of claim 1 wherein the active polypeptide is a surface antigen of a mammalian tumor cell.

4. A hybrid surface protein of claim 1 wherein the active polypeptide is a surface antigen of male sperm.

5. A hybrid surface protein of claim 1 wherein the active polypeptide is an allergen.

6. A hybrid surface protein of claim 1 wherein the active polypeptide includes an antigenic determinant of a surface antigen of a bacteria, virus, parasite or fungus.

7. A hybrid surface protein according to anyone of claims 1 to 6 wherein the anchor sequence is the anchor sequence of a streptococcal M protein.

8. A gene sequence which codes for a hybrid surface protein according to any of claims 1 to 7.

9. A plasmid comprising the gene sequence claimed in claim 8.

10. A chromosome comprising the gene sequence claimed in claim 8.

11. A non-pathogenic gram positive bacteria which expresses a hybrid surface protein according to any of claims 1 to 7, wherein the anchor sequence of said protein is attached to the bacteria.

12. A non-pathogenic gram positive bacteria according to claim 11 wherein the hybrid surface protein is expressed by a plasmid according to claim 9.

13. A non-pathogenic gram positive bacteria according to claim 11 wherein the hybrid surface protein is expressed by a chromosomal gene.

14. A vaccine to protect an animal host against infection by a pathogenic bacteria which comprises a bacteria according to any of claims 11 to 13 and a pharmaceutically acceptable carrier.

15. A plasmid comprising a gene segment for expressing the anchor sequence LPXTGX of a surface antigen normally expressed by a gram positive bacteria together with a polylinker segment which is separated into two sections by a marker.

16. An in-vitro method of testing for an infection which is **characterized by** the presence of antibodies in a body fluid of an animal, said antibodies being characteristic of the infecting organism, said test comprising incubating the body fluid with a non-pathogenic gram positive bacteria according to any of claims 11 to 13, which will react with said antibodies and thereafter, determining if a reaction has taken place.

17. A kit useful for testing to determine the presence of an infection, said infection being **characterized by** the presence of antibodies in a body fluid of an animal, said kit containing a non-pathogenic gram positive bacterium which expresses a hybrid surface protein according to any of claims 11 to 13, which will react with said antibodies.

## Patentansprüche

1. Ein Oberflächenhybridprotein, enthaltend die Ankersequenz LPXTGX eines Oberflächenantigens, das normalerweise durch ein grampositives Bakterium exprimiert wird, gebunden an ein aktives Polypeptid, welches einem menschlichen Wirt zu einem nützlichen Zweck übertragen werden kann.

2. Das Oberflächenhybridprotein nach Anspruch 1, wobei das aktive Polypeptid ein Enzym ist.

3. Ein Oberflächenhybridprotein nach Anspruch 1, wobei das aktive Polypeptid ein Oberflächenantigen einer menschlichen Tumorzelle ist.

4. Ein Oberflächenhybridprotein nach Anspruch 1, wobei das aktive Polypeptid ein Oberflächenantigen eines männlichen Spermiums ist.

5. Ein Oberflächenhybridprotein nach Anspruch 1, wobei das aktive Polypeptid ein Allergen ist.

6. Ein Oberflächenhybridprotein nach Anspruch 1, wobei das aktive Polypeptid eine antigene Determinante eines Oberflächenantigens eines Bakteriums, eines Virus, eines Parasiten oder eines Fungus beinhaltet.

7. Ein Oberflächenhybridprotein gemäß einem der Ansprüche 1 bis 6, wobei die Ankersequenz die Ankersequenz des streptokokkalen M-Proteins ist.

8. Eine Gensequenz, welche für ein Oberflächenhybridprotein gemäß einem der Ansprüche 1 bis 7 kodiert.

9. Ein Plasmid, der die Gensequenz umfaßt, die in Anspruch 8 beansprucht ist.

10. Ein Chromosom, das die Gensequenz umfaßt, die in Anspruch 8 beansprucht ist.

11. Ein nicht-pathogenes grampositives Bakterium, welches ein Oberflächenhybridprotein gemäß einem der Ansprüche 1 bis 7 exprimiert, wobei die Ankersequenz des Proteins an dem Bakterium anhaftet.

12. Ein nicht-patogenes grampositives Bakterium gemäß Anspruch 11, wobei das Oberflächenhybridprotein durch einen Plasmid nach Anspruch 9 exprimiert wird.

13. Ein nicht-pathogenes grampositives Bakterium gemäß Anspruch 11, wobei das Oberflächenhybridprotein durch ein chromosomales Gen exprimiert wird.

14. Ein Impfstoff, zum Schutz eines tierischen Wirts gegen die Infektion durch ein pathogenes Bakterium, welcher ein Bakterium gemäß einem der Ansprüche 11 bis 13 und einen pharmazeutisch akzeptablen Träger umfaßt.

15. Ein Plasmid, das ein Gensegment zum Exprimieren der Ankersequenz LPXTGX eines Oberflächenantigens, welches normalerweise durch ein grampositives Bakterium exprimiert wird, gemeinsam mit einem Polylinkersegment umfaßt, welches durch einen Marker in zwei Abschnitte geteilt wird.

16. Ein in-vitro-Verfahren zum Testen, ob eine Infektion vorliegt, welche **gekennzeichnet ist durch** die Gegenwart von Antikörpern in einer Körperflüssigkeit eines Tieres, wobei die Antikörper charakteristisch für den infizierenden Organismus sind, wobei der Test das Inkubieren der Körperflüssigkeit mit einem nichtpathogenen grampositiven Bakterium gemäß einem der Ansprüche 1 bis 13, welche mit den Antikörpern reagieren werden und danach das Bestimmen, ob eine Reaktion stattgefunden hat, umfaßt.

17. Ein Kit, das nützlich für einen Test zum Bestimmen der Gegenwart einer Infektion ist, wobei die Infektion **dadurch gekennzeichnet ist, daß** Antikörper in einer Körperflüssigkeit eines Tieres vorhanden sind, wobei das Kit ein nicht-pathogenes grampositives Bakterium umfaßt, welches ein Oberflächenhybridprotein gemäß einem der Ansprüche 11 bis 13 exprimiert, welches mit den Antikörpern reagieren wird.

## Revendications

1. Protéine de surface hybride comprenant la séquence d'ancrage LPXTGX d'un antigène de surface normalement exprimé par une bactérie gram-positive fusionnée à un polypeptide actif qui peut être délivrée à un hôte mammifère dans un objectif utile.

2. Protéine de surface hybride selon la revendication 1, dans laquelle le polypeptide actif est une enzyme.

3. Protéine de surface hybride selon la revendication 1, dans laquelle le polypeptide actif est un antigène de surface d'une cellule de tumeur de mammifère.

4. Protéine de surface hybride selon la revendication 1, dans laquelle le polypeptide actif est un antigène de surface de sperme mâle.

5. Protéine de surface hybride selon la revendication 1, dans laquelle le polypeptide actif est un allergène.

6. Protéine de surface hybride selon la revendication 1, dans laquelle le polypeptide actif comprend un déterminant antigénique d'un antigène de surface d'une bactérie, d'un virus, d'un parasite ou d'un champignon.

7. Protéine de surface hybride selon l'une quelconque des revendications 1 à 6, dans laquelle la séquence d'ancrage est la séquence d'ancrage d'une protéine M streptococcique.

8. Séquence de gène qui code une protéine de surface hybride selon l'une quelconque des revendications 1 à 7.

9. Plasmide comprenant la séquence de gène revendiquée dans la revendication 8.

10. Chromosome comprenant la séquence de gène revendiquée dans la revendication 8.

11. Bactérie gram-positive non pathogène qui exprime une protéine de surface hybride selon l'une quelconque des revendications 1 à 7, dans laquelle la séquence d'ancrage de ladite protéine est attachée à la bactérie.

12. Bactérie gram-positive non pathogène selon la revendication 11, dans laquelle la protéine de surface hybride est exprimée par un plasmide selon la revendication 9.

13. Bactérie gram-positive non pathogène selon la revendication 11, dans laquelle la protéine de surface hybride est exprimée par un gène chromosomique.

14. Vaccin pour protéger un hôte animal contre une infection par une bactérie pathogène qui comprend une bactérie selon l'une quelconque des revendications 11 à 13 et un véhicule pharmaceutiquement acceptable.

15. Plasmide comprenant un segment de gène pour exprimer la séquence d'ancrage LPXTGX d'un antigène de surface normalement exprimé par une bactérie gram-positive avec un segment lieur multisites qui est séparé en deux sections par un marqueur.

16. Procédé in vitro de test d'une infection qui se **caractérise par** la présence d'anticorps dans un fluide corporel d'un animal, lesdits anticorps étant caractéristiques de l'organisme infectieux, ledit test comprenant le fait d'incuber le fluide corporel avec une bactérie gram-positive non pathogène selon l'une quelconque des revendications 1 à 13 qui réagira avec lesdits anticorps, et à déterminer ensuite si une réaction a eu lieu.

17. Trousse de test utile pour déterminer la présence d'une infection, ladite infection **se caractérisant par** la présence d'anticorps dans un fluide corporel d'un animal, ladite trousse contenant une bactérie gram-positive non pathogène qui exprime une protéine de surface hybride selon l'une quelconque des revendications 1 à 13, qui réagira avec lesdits anticorps.
